# EUROPEAN PATENT SPECIFICATION

(11) **EP 1 231 946 B1**
(45) Date of publication and mention of the grant of the patent: **12.01.2005**
(21) Application number: 00989879.2
(22) Date of filing: 23.11.2000
(51) Int. Cl.: A61L 15/44, A61K 33/30, A61P 31/04

(54) **MEDICATED DRESSINGS CONTAINING ZINC OXIDE PARTICLES HAVING ANTIMICROBIAL ACTIVITY**
MEDIZINISCHE VERBANDMITTEL ENTHALTEND ZINKOXID MIT ANTIMIKROBIELLER WIRKUNG
PANSEMENTS MEDICAMENTEUX CONTENANT DES PARTICULES D'OXIDE DE ZINC AYANT UNE ACTIVITE ANTIMICROBIALE

(30) Priority: 24.11.1999 GB 9927648
(43) Date of publication of application: 21.08.2002
(73) Proprietor: The Boots Company PLC, Nottingham, Nottinghamshire NG2 3AA (GB); ELEMENTIS UK LIMITED, London EC3R 5AH (GB)
(72) Inventor: GALLEY, Edward The Boots Company Plc, Nottingham, Nottinghamshire NG2 3AA (GB); LAUNDON, Roy, David Elementis UK Ltd, London EC3R 5AH (GB)
(74) Representative: Jones, Stephen Anthony
(86) International application number: PCT/EP2000/011713
(87) International publication number: WO 2001/037888

(56) References cited:
- WO-A-95/04704
- WO-A-99/59540
- GB-A- 2 233 556
- GB-A- 2 269 745
- US-A- 4 486 488
- US-A- 4 685 907
- TICHY S: "TRANSPARENT ZNO FOR SKIN- AND SUNPROTECTION" SOFW-JOURNAL SEIFEN, OELE, FETTE, WACHSE,DE,VERLAG FUR CHEMISCHE INDUSTRIE, H. ZIOLKOWSKY K.G. AUGSBURG, vol. 119, no. 8, 8 June 1993 (1993-06-08), pages 487-490, XP000370086 ISSN: 0942-7694

## Description

This invention teaches the use of a modified form of zinc oxide in medicated dressings as an agent to inhibit microbial growth.

Micro-organisms, present upon the skin exacerbate many minor human afflictions and can cause infection of wounds. It is usual practice to cover such wounds with medical dressings which may contain antimicrobial agents. Efforts have focused upon developing agents which will kill off the micro-organisms, and are suitable for application to the human body. Not only do micro-organisms continuously develop resistance but many successful antimicrobial agents cause burning or irritation to the skin. Thus, there is an enormous need for new antimicrobial agents.

The present invention provides medicated dressings comprising an antimicrobially effective amount of high surface area zinc oxide particles, having a surface area between 30m²/g and 120m²/g and a particle size between 0.3 and 200 µm in diameter. The term "medicated dressing" as used herein is intended to embrace the known forms of medical dressings which include adhesive and non-adhesive bandages, plasters, compresses, poultices and cataplasms which are intended to be applied directly to wounds. The term "medicated dressing" is also intended to embrace medical sheet materials which are intended to provide protection against microbial growth in areas around a wound or the site of a surgical incision. Examples of suitable medical sheet materials include (a) surgical drapes which are used in operating theatres to cover parts of the body around the site being operated on, (b) surgical field dressings, (c) surgical gowns worn by a patient during surgery and (d) liners for use inside casts, for example plaster casts. The presence of an antimicrobial amount of the modified form of zinc oxide in the medical sheet materials described above will minimise the risk of microbial infection of the site of surgery. The zinc oxide particles having a surface area between 30m²/g and 120m²/g and having a particle diameter between 0.3 and 200µm are described hereinafter as "high surface area zinc oxide". The surface area of the material can be determined using laser diffraction. From the surface area, the mean estimated spherical diameter may be calculated. The diameter of the particles is henceforth taken to mean the mean estimated spherical diameter. The surface area of the high surface area zinc oxide particles used with this invention was calculated using a Micromeritics Flavsorb II 2300 BET apparatus. Such a zinc oxide is commercially available from Elementis as Activox C80.

The production of such zinc oxides is well documented.

### WO 95/04704 (Harcros)

This patent discloses a method for the production of zinc oxide in the form of discrete particles which have an average particle size of 0.08µm or less in diameter and a surface area >12.5m²/g. The zinc oxide produced is found to be particularly good for as an additive for scattering/absorbing UV light.

### S. Tichy, SOFW - Journal 119 Jahrgang 8/93

This article teaches a method for producing zinc oxide with a particle size of 0.20 µm and a surface area of 50-150m²/g.

### Liu et al Journal of Materials Science 21 (1986) 3698-3702

This describes another method of producing zinc oxide particles with a diameter of 0.15µm and a surface area of about 50m²/g.

These articles are but a small selection from a large number of articles detailing the manufacture of high surface area zinc oxide particles.

WO 9959540 discloses a topical antimicrobial composition containing high surface area zinc oxide powder. US 4685907 relates to a dressing which incorporates zinc oxide, and a method for preparing such dressings.

Surprisingly, it has been found that high surface area zinc oxide is a potent agent for stopping microbial growth when incorporated into medicated dressings. *In vitro* trials have demonstrated its great efficacy against the micro-organisms responsible for a number of common human afflictions. However, for them to be effective in use, they must be applied to the body.

Many antimicrobial agents are harsh reagents that cause burning and irritation to human skin, and so are unsuitable for application to the human body. High surface area zinc oxide is a mild reagent which does not cause burning and irritation to the skin. Thus it is suitable for incorporation into medicated dressings designed to be applied to human skin.

Because of its high potency and because it does not irritate the skin, high surface area zinc oxide may be included in medicated dressings designed to treat some of the more common human afflictions which are microbial in origin or exacerbated by subsequent microbial growth.

Microbes are commonly taken to mean any organism too small to be seen unless by microscopy. For the purposes of this invention it is further defined to include bacteria and microscopic fungi. Microscopic fungi is defined to include yeasts.

In a preferred embodiment of the present invention , the high surface area zinc oxide is present from 1 to 25%, preferably 3 to 15%, most preferably 4 to 12% by weight of the total composition.

The medicated dressings of the present invention may be any of those known in the art. Suitable medicated dressings are described in British Pharmaceutical Codex 1973. In one embodiment the medicated dressing may be a pad of absorbent material which may be fibrous or foamed into which has been incorporated the high surface area zinc oxide. If the pad is manufactured from natural (for example cotton) or synthetic fibres or synthetic foamed materials, the high surface area zinc oxide may be incorporated during the manufacture of the fibres or foamed material. Alternatively, a liquid composition such as a slurry of high surface area zinc oxide may be applied to the fibres after they have been formed by spraying the liquid composition on to the fibres or by passing the threads through a bath containing the liquid composition. The carrier liquid from the liquid composition may then be removed by drying the fibres which can then be formed into a woven or non-woven pad of absorbent material by methods which are well-known in the art

A suitable material for the manufacture of synthetic fibre for use in the preparation of the pad of absorbent is alginate. Such fibres are well-known to those skilled in the art and may be prepared by passing solutions of sodium or potassium alginate through a bath containing a solution of divalent metal salts (for example calcium salts) to form alginate fibres which can then be spun and formed into absorbent pads of the present invention. The solution of divalent metal ions may also contain high surface area zinc oxide which is incorporated into the alginate fibres as they are formed.

The high surface area zinc oxide may be incorporated into a ready-formed pad by impregnating the pad with a liquid composition containing the high surface area zinc oxide using a spray, bath or doctor blade. The liquid carrier used in such an impregnation may be completely or partially removed after impregnation.

In use pads such as those described above may be placed over the area of skin to be treated and held in place by a bandage or by adhesive backing sheet. The pad may be attached to an adhesive backing sheet which is used to hold the pad on the skin in the manner of the known sticking plasters. The backing sheet may be a woven fabric for example a cotton and/or viscose fabric; a non-woven textile for example of non-woven synthetic fibres or a film of synthetic plastic material for example polyethylene which may be perforated. The backing sheet may be permeable to allow the passage therethrough of water vapour, air and bacteria; semi-permeable to allow the passage therethrough of water vapour and air or occlusive to prevent the passage therethrough of water vapour, air and bacteria. The backing sheet may be coated or partially impregnated with an adhesive. Suitable adhesives are well known in the art and include natural elastomers such as para or crêpe rubber or synthetic elastomers such as isobutylene/alkyl-acrylate polymers. If it is desired to treat the area of skin which comes into contact with the adhesive then the high surface zinc oxide may be incorporated into the adhesive as it is coated onto the backing sheet. Known adhesives for adhesive medical dressings may contain zinc oxide BP. The zinc oxide BP in such dressings may be partially or completely replaced by high surface area zinc oxide to give medical dressings of the present invention. From 10 to 100% of the zinc oxide BP may be replaced by the high surface area zinc oxide.

The medicated dressings of the present invention may contain other components. Examples of suitable components include:-
a) antibacterials such as dichlorobenzyl alcohol, triclosan, chlorhexidine digluconate and salicylic acid;
b) oil absorbers such as silica;
c) alcohols such as denatured ethanol, isopropyl alcohol;
d) humectants such as panthenol, butylene glycol, glycerin and propylene glycol;
e) preservatives such as methyldibromo glutaronitrile, phenoxyethanol, magnesium chloride, magnesium nitrate, methylchloroisothiazolinone, methylisothiazolinone or any paraben, for example such as methylparaben, ethyl paraben and propyl paraben;
f) plasticisers
g) antioxidants

The antimicrobial action of high surface area zinc oxide is illustrated by the following methods given below. The material was tested against three bacteria, implicated in dandruff and acne; *P. ovale* and *S. aureus..* In the following experiments the zinc oxide used has a surface area greater than 90m²/g and an average diameter of 10.47 µm.

### ANTIMICROBIAL ACTION AGAINST P. OVALE and S. AUREUS

### Method

The high surface area zinc oxide was dispersed at a maximum concentration of 25% in acetone. Two fold dilutions were prepared in acetone and aliquots of each dilution added to the appropriate agars to give a range of concentrations from 2.5% to 0.16%. Test plates and "no product" controls containing water only were streaked with the test organisms. These were then incubated as appropriate for three days.

The *P.ovale* was incubated on *P.ovale* growth medium, made using:-

| Material | Quantity |
|---|---|
| Malt extract agar | 60g |
| Ox bile - desiccated | 20g |
| Tween 40 | 10.0ml |
| Glycerol mono-oleate | 2.5ml |
| Water | to 1 litre |

This was then incubated aerobically at 37°C for three days.

*S.aureus* was tested on Tryptone Soya Agar which was incubated aerobically for 37°C for two days.

### Results

| | **Water Control** | **Acetone Control** | **High Surface Area Zinc Oxide Concentrations** | | | | |
|---|---|---|---|---|---|---|---|
| | | | 2.5% | 1.25% | 0.6% | 0.31% | 0.16% |
| *P.ovale* | Growth | Growth | No Growth | No Growth | No Growth | No Growth | No Growth |
| *S.aureus* | Growth | Growth | No Growth | No Growth | No Growth | No Growth | No Growth |

Inhibition of growth was observed at the lowest concentration tested (0.16% in agar) for both test organisms.

### Experiment 2

Determination of Minimum Inhibitory Concentrations (MIC) for High Surface Area Zinc Oxide.

### Method

The MIC of high surface area zinc oxide was determined against *P. ovale* and *S. aureus* using Zinc Oxide BP as a control. MIC is the minimum concentration at which the active material will inhibit the growth of the microorganisms to be tested. The control of Zinc Oxide BP, and the test, high surface area zinc oxide, were suspended in acetone at 12.5% and aliquots were added to the agar to give a range of concentrations from 6250ppm to 98ppm.

The *P.ovale* was incubated on *P.ovale* growth medium, made using:-

| Material | Quantity |
|---|---|
| Malt extract agar | 60g |
| Ox bile - desiccated | 20g |
| Tween 40 | 10.0ml |
| Glycerol mono-oleate | 2.5ml |
| Water | to 1 litre |

This was then incubated aerobically at 37°C for three days.

*S.aureus* was tested on Tryptone Soya Agar which was incubated aerobically at 37°C for two days.

### Results

**Table 2**

| **Microorganism** | **High Surface Area Zinc Oxide MIC** | **Zinc Oxide BP Control MIC** |
|---|---|---|
| *P. ovale* | 1562 ppm | 3125 ppm |
| *S. aureus* | 390 ppm | >6250 ppm |

From this we can see that high surface area zinc oxide inhibits the growth of *P.ovale* and *S.aureus* at a lower concentration than the Zinc Oxide BP control..

This means that the high surface area zinc oxide inhibits the growth of these organisms better than the Zinc Oxide control. These results were obtained using the neat raw materials.

The invention is further disclosed by way of the following, non limiting examples. Unless otherwise stated, the zinc oxide used in the following formulations has a surface area greater than 90m²/g and the particles have an average diameter of 10.47 µm.

### Example 1

A pad of cotton fabric impregnated with high surface area zinc oxide (10% by weight of the pad) is placed on to centre of a backing sheet which is portion of woven cotton tape to which a rubber adhesive had been applied. A protective sheet is placed over the pad and the face of the tape to which the adhesive had been applied to keep the resulting permeable medicated dressing clean before use. The dressing was packed in a non-permeable sachet to preserve it in a sterile condition prior to use.

### Example 2

A medicated dressing is manufactured as described in Example 1 except that the rubber adhesive is replaced by an isobutylene/alkyl-acrylate polymer.

### Example 3

A medicated dressing is manufactured as described in Example 2 except that the backing sheet is replaced by a sheet of polyethylene to give an occlusive medicated dressing.

### Example 4

A medicated dressing is manufactured as described in Example 2 except that the backing sheet is replaced by a sheet of polyethylene which has been perforated to give to give a semi-permeable medicated dressing.

## Claims

1. Medicated dressings in the form of adhesive bandages, non-adhesive bandages, plasters, compresses, poultices, cataplasms, surgical drapes, surgical field dressings, surgical gowns or liners for use inside casts, comprising an antimicrobially effective amount of high surface area zinc oxide particles, having a surface area between 30m²/g and 120m²/g and a particle size between 0.3 and 200 µm in diameter.

2. A medicated dressing as claimed in claim 1 where the high surface area zinc oxide is present from 1 to 25% by weight of the total composition.

3. A medicated dressing as daimed in claim 1 where the high surface area zinc oxide is present from 3 to 15% by weight of the total composition.

4. Medicated dressings as claimed in any preceding claim in the form of a fibrous or foamed pad of absorbent material into which the high surface area zinc oxide has been incorporated.

5. Medicated dressings as claimed in claim 4 wherein the high surface area zinc oxide is incorporated into the pad of absorbent material during the manufacture of the fibres from which the pad is made or is impregnated into the pad after the pad has been manufactured.

6. Medicated dressings as claimed in claim 5 wherein the fibres are cotton or alginate.

## Patentansprüche

1. Medizinische Wundauflagen in Form von klebenden Bandagen, nichtklebenden Bandagen, Pflastern, Kompressen, Breiumschlägen, Kataplasmen, Verbandtüchem, Operationsdecken, Operationskitteln oder Operationshandschuhen für die Verwendung im Innern von Gipsverbänden, die eine anti-mikrobisch wirksame Menge von Zinkoxid-Partikeln mit einer hohen spezifischen Oberfläche umfasst und eine spezifische Oberfläche zwischen 30 m²/g und 120 m²/g und eine Partikelgröße zwischen 0,3 und 200 µm im Durchmesser besitzen.

2. Eine medizinische Wundauflage nach Anspruch 1, wobei das Zinkoxid mit hoher spezifischer Oberfläche in 1 bis 25 Gew.% der Gesamtzusammensetzung vorhanden ist.

3. Eine medizinische Wundauflage nach Anspruch 1, wobei das Zinkoxid mit hoher spezifischer Oberfläche in 3 bis 15 Gew.% der Gesamtzusammensetzung vorhanden ist.

4. Medizinische Wundauflage nach einem der vorhergehenden Ansprüche in der Form eines Faser- oder Schaumstoff-Wattebausches eines absorbierenden Materials, in den das Zinkoxid mit einer hohen spezifischen Oberfläche aufgenommen wurde.

5. Medizinische Wundauflage nach Anspruch 4, wobei das Zinkoxid mit einer hohen spezifischen Oberfläche, während der Herstellung der Fasern, aus denen der Wattebausch gemacht ist, in dem Wattebausch aufgenommen wird oder nachdem der Wattebausch hergestellt ist, in den Wattebausch imprägniert wird.

6. Medizinische Wundauflage nach Anspruch 5, wobei die Fasern aus Baumwolle oder Alginat bestehen.

## Revendications

1. Bandages thérapeutiques sous la forme de pansements adhésifs, pansements non-adhésifs, sparadrap, compresses, sinapismes, cataplasmes, drapage opératoire, objets de pansement, blouses chirurgicales ou fonds protecteurs pour l'intérieur des plâtres, comprenant une quantité effective antimicrobienne de particules d'oxyde de zinc de grande surface spécifique, d'une surface spécifique située entre 30m²/g et 120m²/g et une taille de particule située entre 0,3 et 200 µm de diamètre.

2. Un bandage thérapeutique selon la revendication 1 où l'oxyde de zinc de grande surface spécifique représente de 1 à 25% du poids de la composition totale.

3. Un bandage thérapeutique selon la revendication 1 où l'oxyde de zinc de grande surface spécifique représente de 3 à 15% du poids de la composition totale.

4. Des bandages thérapeutiques selon l'une quelconque des revendications précédentes sous la forme d'un tampon fibreux ou d'un coussinet mousse en matériau absorbant dans lequel l'oxyde de zinc de grande surface spécifique a été incorporé.

5. Des bandages thérapeutiques selon la revendication 4 dans lesquels l'oxyde de zinc de grande surface spécifique est incorporé au tampon fait de matériau absorbant au cours de la fabrication des fibres composant le tampon ou est imprégné dans le tampon après sa fabrication.

6. Des bandages thérapeutiques selon la revendication 5 dans lesquels les fibres sont en coton ou en alginate.
